# EUROPEAN PATENT SPECIFICATION

(11) **EP 4 134 119 B1**
(45) Date of publication and mention of the grant of the patent: **15.07.2026**
(21) Application number: 21818299.6
(22) Date of filing: 26.03.2021
(51) Int. Cl.: A61M 25/00

(54) **CATHETER**
KATHETER
CATHÉTER

(30) Priority: 01.06.2020 JP 2020095742; 30.11.2020 JP 2020197870
(43) Date of publication of application: 15.02.2023
(73) Proprietor: TERUMO Kabushiki Kaisha, Tokyo 151-0072 (JP)
(72) Inventor: GUIMARAES Marcelo, South Carolina 29425 (US); OKAMURA Ryo, Ashigarakami-gun, Kanagawa 2590151 (JP); YASUNAGA Mitsuteru, Fujinomiya-shi, Shizuoka 418-0015 (JP)
(74) Representative: Casalonga
(86) International application number: PCT/JP2021/012976
(87) International publication number: WO 2021/246030

(56) References cited:
- EP-A1- 2 016 969
- EP-A2- 0 829 270
- WO-A1-2006/019640
- WO-A1-2014/197962
- WO-A2-2007/030264
- JP-A- 2020 151 473
- JP-A- H11 206 886
- US-A- 5 820 592
- US-A- 6 039 723
- US-A1- 2007 112 405

## Description

### Technical Field

The present invention relates to a catheter for uterine artery and prostate artery engagement.
From US 2007/112405 A1, EP 0829 270 A2, EP 2 016 969 A1, US 6 039 723 A and US 5 820 592 A, there are already known catheters for coronary artery engagement comprising:
a tube shaped body communicating from a proximal end to a distal end,
wherein the tube shaped body includes, at a distal portion thereof, a shaped portion shaped in a manner of being bent on substantially the same plane,
the shaped portion includes:
   a proximal bent portion bent to define a first angle
   a distal bent portion defining a second angle distal (θ2) of the proximal bent portion and bent to the same side as the proximal bent portion;
   an intermediate linear portion that is linear and is disposed between the proximal bent portion and the distal bent portion; and
   a distal linear portion that is linear and is disposed distal of the distal bent portion , and
a first minimum curvature radius which is a smallest curvature radius of an axis of the proximal bent portion is no less than 3 times and no more than 11 times a second minimum curvature radius which is a smallest curvature radius of an axis of the distal bent portion.

None of these prior art document considers use a catheter for uterine artery or prostate artery engagement.

### Background Art

Currently, intervention is performed in which treatment of a lesion area of a body is performed by an elongated catheter inserted into a blood vessel from a hole opened in a skin.

For example, PTL 1 discloses percutaneous coronary intervention (PCI) of performing treatment of a coronary artery by trans radical intervention (TRI) of inserting a catheter from a radial artery of a wrist. Further, PTL 2 discloses uterine fibroid embolization (UFE) of embolizing a uterine artery for treatment of a uterine fibroid by trans femoral intervention (TFI) of inserting a catheter from a femoral artery.

### Citation List

### Patent Literature

PTL 1: US Patent No. 6355026
PTL 2: US Patent No. 7422581

### Summary of Invention

### Technical Problem

Inserting the catheter from the radial artery reduces a physical burden on a patient, but the catheter that can be inserted is thinner. In addition, in order to cause the catheter to reach the uterine artery or the prostate artery from the radial artery, it is necessary to move the catheter along a long path while crossing a plurality of branches of blood vessels. Therefore, for example, when a catheter for PCI is used, it is difficult to cause the catheter to reach a target position.

The invention has been made in order to solve the above-described technical problems, and an object of the invention is to provide a catheter that can easily reach and be engaged with a uterine artery or a prostate artery from a radial artery.

### Solution to Problem

In order to solve the above technical problem, the invention provides a catheter for uterine artery or prostate artery engagement according to claim 1.

### Advantageous Effect of Invention

In the catheter configured as described above, the proximal bent portion is in contact with the internal iliac artery, and the distal bent portion that is sharply bent to the same direction as the proximal bent portion with a curvature radius smaller than that of the proximal bent portion and/or the distal linear portion distal of the distal bent portion are easily directed toward the uterine artery (or prostate artery) and engaged with the uterine artery (or prostate artery). Therefore, it is easy for the catheter to reach the uterine artery or prostate artery)from the radial artery and be engaged with the uterine artery (or prostate artery).

The first minimum curvature radius may be 25 mm, the second minimum curvature radius may be 5 mm, the first minimum curvature radius may be 5 times the second minimum curvature radius, a length along an axis of the intermediate linear portion may be 16 mm, and a length along an axis of the distal linear portion may be 3 mm. Therefore, it is easy for the catheter to reach and be engaged with the uterine artery or the prostate artery from the radial artery.

The catheter according to the invention may be employed in a treatment method using a catheter including a tube shaped body communicating from a proximal end to a distal end, and the treatment method includes: inserting, into a blood vessel from a radial artery, the catheter in which the tube shaped body includes, at a distal portion thereof, a shaped portion shaped in a manner of being bent on substantially the same plane, the shaped portion includes a proximal bent portion defining a first angle, a distal bent portion defining a second angle distal of the proximal bent portion and bent to the same side as the proximal bent portion, an intermediate linear portion that is linear and is disposed between the proximal bent portion and the distal bent portion, and a distal linear portion that is linear and is disposed distal of the distal bent portion, and a first minimum curvature radius which is a smallest curvature radius of an axis of the proximal bent portion is no less than 3 times and no more than 11 times a second minimum curvature radius which is a smallest curvature radius of an axis of the distal bent portion; and engaging the shaped portion with a blood vessel more peripheral than an internal iliac artery.

In the treatment method configured as described above and not falling under the scope of the claims, the proximal bent portion is brought into contact with the internal iliac artery, and the distal bent portion that is bent more sharply than the proximal bent portion in the same direction as the proximal bent portion and/or the distal linear portion distal of the distal bent portion are easily directed toward and engaged with the blood vessel more peripheral than the internal iliac artery. Therefore, it is easy for a surgeon to perform treatment in a favorable manner by causing the catheter to reach and be engaged with the blood vessel more peripheral than the internal iliac artery from the radial artery.

In this treatment method not falling under the scope of the claims, the radial artery into which the catheter is to be inserted may be a distal radial artery. Accordingly, it is easy for the surgeon to perform the treatment in a favorable manner by causing the catheter to reach and be engaged with the blood vessel more peripheral than the internal iliac artery in a less invasive manner than in a case of inserting the catheter from a conventional radial artery.

The blood vessel more peripheral than the internal iliac artery to be engaged with the shaped portion may be a prostate artery. Accordingly, the surgeon can treat the prostate in a favorable manner by using the catheter inserted into the blood vessel from the radial artery.

The blood vessel more peripheral than the internal iliac artery to be engaged with the shaped portion may be a uterine artery. Accordingly, the surgeon can treat a uterus in a favorable manner by using the catheter inserted into the blood vessel from the radial artery.

A treatment target may be an enlarged prostate due to the benign prostatic hyperplasia. Accordingly, the surgeon can treat the benign prostatic hyperplasia in a favorable manner by using the catheter inserted into the blood vessel from the radial artery.

A treatment target may be a uterine fibroid. Accordingly, the surgeon can treat the uterine fibroid in a favorable manner by using the catheter inserted into the blood vessel from the radial artery.

### Brief Description of Drawings

FIG. 1 is a schematic diagram showing an arrangement of a catheter in a blood vessel.
FIG. 2 is a plan view showing a distal portion of the catheter according to the embodiment.
FIG. 3 is a schematic diagram showing a state in which the catheter is inserted from a left radial artery, a proximal bent portion is attached to a left subclavian artery, a distal bent portion is disposed in an aorta, and a distal linear portion and a distal opening portion are directed to a descending aorta.
FIG. 4 is a schematic diagram showing a state in which the catheter is inserted into a common iliac artery and an internal iliac artery.
FIG. 5 is a schematic diagram showing a state in which the catheter is engaged with a uterine artery.
FIG. 6 is a schematic view showing another example of the state in which the catheter is engaged with the uterine artery.
FIG. 7 is a schematic diagram showing a state in which the catheter is engaged with a prostate artery.

### Description of Embodiments

An embodiment according to the invention will be described hereinafter with reference to the drawings. Note that for convenience of explanation, dimensions in the drawings may be exaggerated and may be different from actual dimensions. Further, in the present specification and the drawings, structural elements that have substantially the same function are denoted with the same reference numerals, and repeated explanation of these structural elements is omitted. In the present description, a side to be inserted into a blood vessel is referred to as a "distal side", and a side to be operated is referred to as a "proximal side".

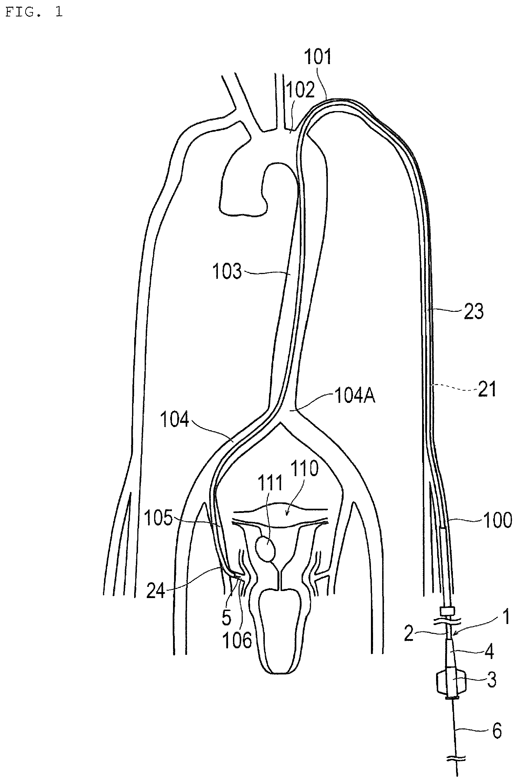

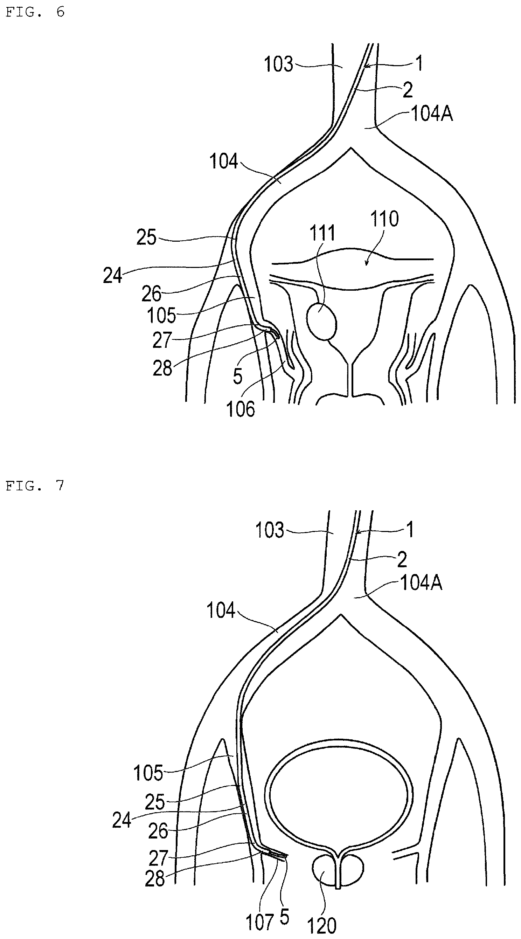

As shown in FIGS. 1 and 6, a catheter 1 according to the present embodiment is a catheter to be engaged with a uterine artery 106 or a prostate artery 107. The catheter 1 is inserted into a blood vessel from a radial artery 100 of an arm of a patient and engaged with the uterine artery 106 or the prostate artery 107. The catheter 1 may be a so-called guiding catheter, an angiographic catheter, a guide wire support catheter, or a microcatheter. In addition, the catheter 1 may be a combination of a guiding catheter and a microcatheter that is inserted into an inner tube of the guiding catheter and is longer than the guiding catheter.

As shown in FIG. 1, the catheter 1 includes a tube shaped body 2, a hub 3 disposed on a proximal side of the tube shaped body 2, and an anti-kink protector 4.

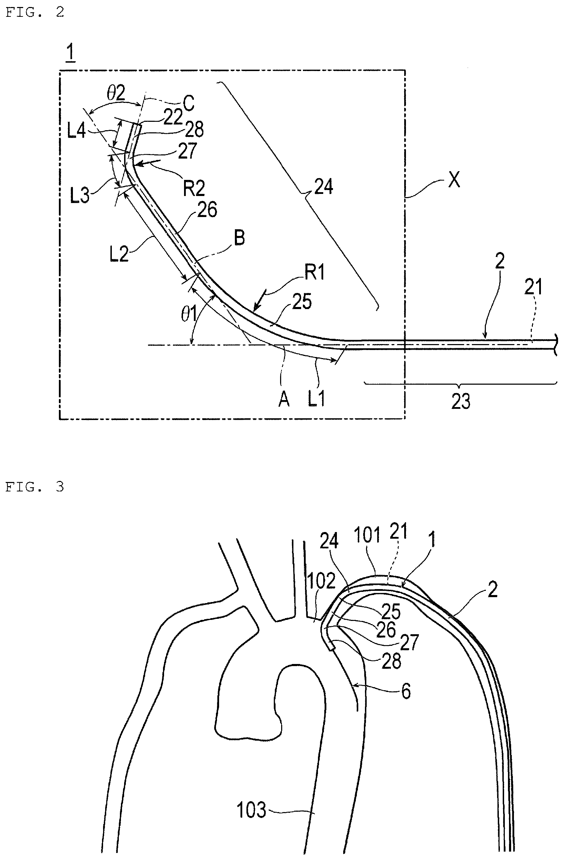

As shown in FIGS. 1 and 2, the tube shaped body 2 is elongated and flexible. The tube shaped body 2 is formed with a lumen 21 extending from a proximal end to a distal end in a substantially central portion thereof.

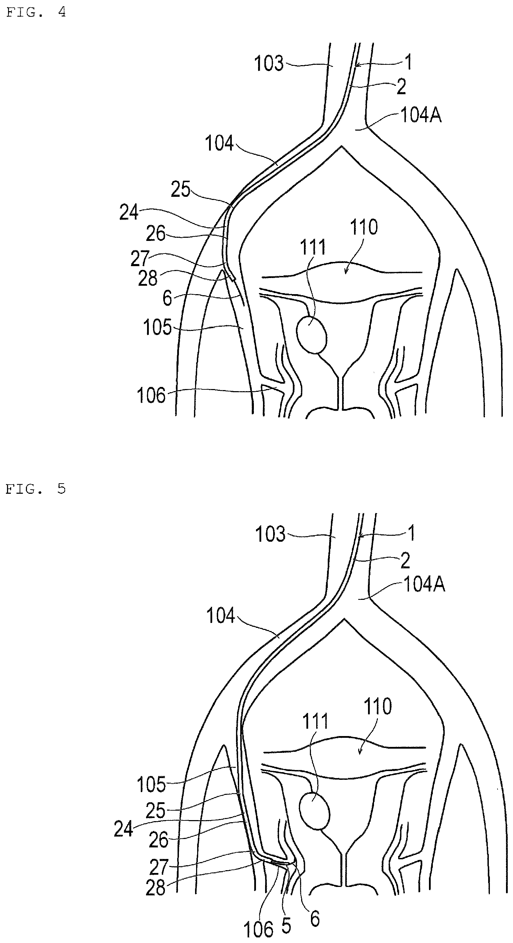

The hub 3 is formed with a passage communicating with the lumen 21. The hub 3 can be used to insert or remove, for example, a guide wire 6, a treatment catheter 5 (see FIG. 5), or the like. In addition, the hub 3 can be used to inject various liquids such as an X-ray contrast agent, a drug solution, and a physiological salt solution.

The anti-kink protector 4 is made of an elastic material. The anti-kink protector 4 covers a portion at which the tube shaped body 2 and the hub 3 are coupled to each other. Accordingly, the anti-kink protector 4 prevents the tube shaped body 2 from being bent or kink in the vicinity of the portion.

Next, the tube shaped body 2 will be described in detail. The tube shaped body 2 includes a substantially linear main body portion 23 and a shaped portion 24 shaped in a bent state. An axis of the shaped portion 24 is located on the same plane X, and the shaped portion 24 is curved on substantially the same plane X. The axis means a line passing through a center of the tube shaped body 2, and means a central axis when the tube shaped body 2 is concentric circles. The shaped portion 24 includes a proximal bent portion 25, an intermediate linear portion 26, a distal bent portion 27, and a distal linear portion 28. The proximal bent portion 25 extends in a distal direction from a distal end of the main body portion 23 and is bent in the plane X. The intermediate linear portion 26 linearly extends in the distal direction from a distal end of the proximal bent portion 25. The distal bent portion 27 extends in the distal direction from a distal end of the intermediate linear portion 26 and is bent in the same direction as the proximal bent portion 25 in the plane X. The distal linear portion 28 linearly extends in the distal direction from a distal end of the distal bent portion 27. Note that the phrase "being curved on substantially the same plane X" may include not only being curved on the same plane X, but also a case in which a distal portion of the catheter 1 slightly protrudes from the plane X to such an extent that the same effect can be practically exhibited.

A line passing through the axis of the main body portion 23 is defined as a first axis A. A line passing through the axis of the intermediate linear portion 26 is defined as a second axis B. A line passing through the axis of the distal linear portion 28 is defined as a third axis C.

An angle formed by the second axis B on the distal side with respect to the first axis A is defined as a first angle θ1. The first angle θ1 is an amount of change in a direction of the axis of the tube shaped body 2 in the proximal bent portion 25. That is, the proximal bent portion 25 defines the first angle θ1. When the first angle θ1 is 0°, the second axis B coincides with the first axis A, and when the first angle θ1 is 180°, the second axis B is folded back in an opposite direction with respect to the first axis A. A curvature radius of an axis of the proximal bent portion 25 may be constant throughout the entire proximal bent portion 25, or may vary depending on portions. In the proximal bent portion 25, a first minimum curvature radius R1 of a portion (a portion that is bent most sharply) having a smallest curvature radius of the axis is not particularly limited, and is preferably 15 mm to 50 mm, and most preferably 25 mm. The first angle θ1 is not particularly limited, and is preferably 15° to 70°, and most preferably 55°.

An angle formed by the third axis C on the distal side with respect to the second axis B is defined as a second angle θ2. The second angle θ2 is an amount of change in the direction of the axis of the tube shaped body 2 in the distal bent portion 27. That is, the distal bent portion 27 defines the second angle θ2. When the second angle θ2 is 0°, the third axis C coincides with the second axis B, and when the second angle θ2 is 180°, the third axis C is folded back in an opposite direction with respect to the second axis B. A curvature radius of an axis of the distal bent portion 27 may be constant throughout the entire distal bent portion 27, or may vary depending on portions. In the distal bent portion 27, a second minimum curvature radius R2 of a portion having a smallest curvature radius of the axis (a portion that is bent most sharply) is not particularly limited, and is preferably 1 mm to 10 mm, and most preferably 5 mm. The second minimum curvature radius R2 is smaller than the first minimum curvature radius R1. The first minimum curvature radius R1 is preferably 3 times or more and 11 times or less the second minimum curvature radius R2, and most preferably 5 times the second minimum curvature radius R2. The second angle θ2 is not particularly limited, and is preferably 30° to 70°, and most preferably 50°.

A length L1 along the axis of the proximal bent portion 25 is not particularly limited, and is, for example, 1 mm to 100 mm. For example, the length L1 along the axis of the proximal bent portion 25 is 24 mm.

A length L2 along the axis of the intermediate linear portion 26 is not particularly limited, and is preferably 1 mm to 100 mm, and most preferably 16 mm.

A length L3 along the axis of the distal bent portion 27 is 1 mm to 10 mm, and preferably 4 mm.

A length L4 of the distal linear portion 28 is not particularly limited, and is preferably 0.5 mm to 50 mm, and most preferably 3 mm.

In the tube shaped body 2, a reinforcement body (not shown) may be embedded in a range from the proximal end to a predetermined position in the distal direction. The reinforcement body is formed by braiding a plurality of wires into a tubular shape. A position of a most distal end of the reinforcement body is not particularly limited, and is disposed in the shaped portion 24 or the main body portion 23. A flexible portion not provided with the reinforcement body is provided distal of a reinforcement portion in which the reinforcement body is embedded. A length of the flexible portion is preferably 500 mm or less, and more preferably 10 to 300 mm. For example, in a case of a catheter 1 of 4 Fr, the length of the flexible portion is 150 mm. In a case of a catheter 1 of 5 Fr, the length of the flexible portion is 150 mm.

The catheter 1 is coated with a lubricating coating in a proximal direction along an axis from a distal end opening portion 22. The lubricating coating is coated in the entire length of the catheter 1, and is coated in a range of 500 mm or less, more preferably 400 mm or less in the proximal direction along the axis from the distal end opening portion 22. Therefore, a surgeon can deeply insert the catheter 1 into the uterine artery 106. Examples of the constituent material of the lubricating coating include: a copolymer including an epoxy group-containing monomer such as glycidyl acrylate, glycidyl methacrylate, 3,4-epoxycyclohexylmethyl acrylate, 3,4-epoxycyclohexylmethyl methacrylate, β-methylglycidyl methacrylate, and allyl glycidyl ether, and a hydrophilic monomer such as N-methylacrylamide, N,N-dimethylacrylamide, and acrylamide; a (co)polymer including the hydrophilic monomer; a cellulose-based polymer material such as hydroxypropyl cellulose and carboxymethyl cellulose; a polysaccharide; a polyvinyl alcohol; a methyl vinyl ether-maleic anhydride copolymer; a water-soluble polyamide; poly(2-hydroxyethyl (meth)acrylate); polyethylene glycol; polyacrylamide; and polyvinyl pyrrolidone.

An outer diameter of the tube shaped body 2 is preferably 1 mm (3 Fr) to 2.5 mm (6 Fr), and more preferably 1.3 mm (4 Fr) to 1.8 mm (5 Fr). An effective length of the tube shaped body 2 is preferably 1300 mm to 1600 mm. It is preferable that a dimension of the tube shaped body 2 can be appropriately selected depending on the patient, a blood vessel to which the catheter 1 is to be introduced, or the like. For example, when the catheter 1 is introduced from the conventional radial artery 100 of a small-sized female, the effective length of the tube shaped body 2 is preferably relatively short. When the catheter 1 is introduced from a distal radial artery of a large-sized male, the effective length of the tube shaped body 2 is preferably relatively long. The effective length is a length of a portion that can be inserted into a blood vessel, a sheath, or the like. In the present embodiment, the effective length is a length from a most distal end of the anti-kink protector 4 to a most distal end of the tube shaped body 2. In the present embodiment, the effective length of the catheter 1 for the uterine artery is 1350 mm, the effective length of the catheter 1 for the prostate artery is 1500 mm, and a length of the lubricating coating is 400 mm from the distal end.

A distal tip that is more flexible than the proximal side may be disposed at the distal portion of the tube shaped body 2. The distal tip is made of a highly flexible material such as a rubber material.

The number of layers constituting the tube shaped body 2, a constituent material of each layer, presence or absence of the reinforcement body, and the like may be different depending on a position in a longitudinal direction of the tube shaped body 2.

Examples of the constituent material of the tube shaped body 2 include various thermoplastic elastomers such as styrene-based, polyolefin-based, polyurethane-based, polyester-based, polyamide-based, polybutadiene-based, transpolyisoprene-based, fluororubber-based, and chlorinated polyethylene-based elastomers, polyetherketone, and polyimide-based ones, and a combination (polymer alloy, polymer blend, laminate, and the like) of one or two or more of above materials can be used. A low-friction material may be disposed on an inner peripheral surface of the tube shaped body 2 so that the guide wire 6 or another catheter may be easily inserted into the lumen 21. The low-friction material is, for example, a fluorine-based resin material such as polytetrafluoroethylene (PTFE). The tube shaped body 2 may contain an X-ray imaging material.

Next, a method (not claimed) of using the catheter 1 according to the invention will be described.

As shown in FIG. 1, the catheter 1 is inserted into a blood vessel from the radial artery 100, passes through a brachial artery and a subclavian artery 101, and is inserted into a descending aorta 103. The catheter 1 is further inserted into a common iliac artery 104 through an aortic iliac artery bifurcated portion 104A of the common iliac artery 104, passes through an internal iliac artery 105, and is engaged with a blood vessel more peripheral than the internal iliac artery 105. The radial artery 100 to which the catheter 1 is to be introduced is, for example, a distal radial artery, a conventional radial artery, a sniff box radial artery, or the like. Here, a case in which the patient is a female and the catheter 1 is inserted into an artery of a left arm to treat a uterine fibroid 111 of a uterus 110 will be described as an example. Note that the catheter 1 may be inserted from the radial artery 100 of a right arm.

In a procedure, the surgeon inserts the guide wire 6 into the radial artery 100. Next, the surgeon pushes the catheter 1 in which the guide wire 6 is accommodated in the lumen 21 forward along the guide wire 6. Normally, a distal end of the guide wire 6 precedes the distal end of the catheter 1. Therefore, the shaped portion 24 of the catheter 1 is deformed by the guide wire 6 in the lumen 21 into a shape closer to a line than an original shape thereof. The shape closer to a line than the original shape of the shaped portion 24 is not limited to a linear shape, and may be a bent shape.

As shown in FIG. 3, the guide wire 6 and the catheter 1 pass through the subclavian artery 101 and advance to an aortic arch 102. At a portion at which the subclavian artery 101 is connected to the aortic arch 102, the catheter 1 needs to be bent greatly in order to move toward the descending aorta 103. In order to cope with this need, for example, the surgeon can temporarily retract the guide wire 6 and accommodate the guide wire 6 in the lumen 21 of the catheter 1. A shape of a portion of the catheter 1 into which the guide wire 6 is not inserted is not limited by the guide wire 6. Therefore, a portion of the shaped portion 24 into which the guide wire 6 is not inserted attempts to restore an original bent shape. When the guide wire 6 retreats toward the proximal side from the shaped portion 24, the entire shaped portion 24 is restored to the original bent shape. Note that the distal end of the guide wire 6 may be positioned inside the shaped portion 24. In this state, the distal bent portion 27 is sharply bent to the same side as the proximal bent portion 25 with a curvature radius smaller than that of the proximal bent portion 25. Further, since the shaped portion 24 in the invention is smaller than a shaped portion of a catheter for coronary artery treatment, a most distal end of the catheter 1 is easily directed to the descending aorta 103, not to an ascending aorta. The surgeon can advance the distal end of the catheter 1 from the aortic arch 102 toward the descending aorta 103 by using bending of the shaped portion 24. Thereafter, the surgeon causes the guide wire 6 to protrude from the catheter 1 toward the distal side. Accordingly, the guide wire 6 can easily advance to the descending aorta 103. Subsequently, the surgeon pushes the catheter 1 forward along the guide wire 6. Accordingly, the catheter 1 can easily advance from the aortic arch 102 to the descending aorta 103. The catheter 1 moves to a peripheral side (a side close to a lower limb) in the descending aorta 103 and reaches the vicinity of the aortic iliac artery bifurcated portion 104A. Note that the surgeon may move the catheter 1 toward the peripheral side in the descending aorta 103 without causing the guide wire 6 to protrude from the catheter 1 toward the distal side.

After the catheter 1 reaches the vicinity of the aortic iliac artery bifurcated portion 104A, the surgeon retracts the guide wire 6 as necessary to accommodate the guide wire 6 in the lumen 21 of the catheter 1. Accordingly, the portion of the shaped portion 24 into which the guide wire 6 is not inserted is restored to the original bent shape. The surgeon can use the bending of the shaped portion 24 to direct the distal end of the catheter 1 toward a common iliac artery on a right or left side through the aortic iliac artery bifurcated portion 104A, thereby easily selecting the common iliac artery 104 on a desired side and inserting the distal end therein.

Thereafter, the surgeon causes the guide wire 6 to protrude from the catheter 1 toward the distal side. Accordingly, as shown in FIG. 4, the catheter 1 can easily advance through the common iliac artery 104. Note that the surgeon may move the catheter 1 toward the peripheral side in the common iliac artery 104 without causing the guide wire 6 to protrude from the catheter 1 toward the distal side. The catheter 1 moves through the common iliac artery 104 to the peripheral side and reaches the vicinity of an entrance of the internal iliac artery 105.

After the guide wire 6 and the catheter 1 reach the vicinity of the entrance of the internal iliac artery 105, the surgeon retracts the guide wire 6 as necessary to accommodate the guide wire 6 in the lumen 21 of the catheter 1. Accordingly, the portion of the shaped portion 24 into which the guide wire 6 is not inserted attempts to restore the original bent shape. In this state, the distal bent portion 27 is sharply bent to the same side as the proximal bent portion 25 with the curvature radius smaller than that of the proximal bent portion 25. Therefore, the most distal end of the catheter 1 is likely to be directed to the internal iliac artery 105 bifurcating from the common iliac artery 104. The surgeon can use the bending of the shaped portion 24 to direct the distal end of the catheter 1 to the entrance of the internal iliac artery 105 and easily insert the distal end without damaging the internal iliac artery 105, which has an inner diameter smaller than that of the common iliac artery 104.

Thereafter, the surgeon causes the guide wire 6 to protrude from the catheter 1 toward the distal side. Accordingly, the catheter 1 can easily advance through the internal iliac artery 105. Note that the surgeon may move the catheter 1 toward a peripheral side in the internal iliac artery 105 without causing the guide wire 6 to protrude from the catheter 1 toward the distal side. The catheter 1 moves through the internal iliac artery 105 to the peripheral side and reaches the vicinity of an entrance of the uterine artery 106.

After the guide wire 6 and the catheter 1 reach the vicinity of the entrance of the uterine artery 106, the surgeon retracts the guide wire 6 as necessary to accommodate the guide wire 6 in the lumen 21 of the catheter 1. Accordingly, as shown in FIG. 5, the portion of the shaped portion 24 into which the guide wire 6 is not inserted attempts to restore the original bent shape. The surgeon can use the bending of the shaped portion 24 to direct the distal end of the catheter 1 to the entrance of the uterine artery 106, and to easily insert the distal end into the uterine artery 106. In this state, the distal bent portion 27 is sharply bent to the same side as the proximal bent portion 25 with the curvature radius smaller than that of the proximal bent portion 25. Therefore, the most distal end of the catheter 1 is more likely to be directed to the uterine artery 106 bifurcating from the internal iliac artery 105, which is thinner than the internal iliac artery 105. Therefore, the distal bent portion 27 and/or the distal linear portion 28 of the catheter 1 are in contact with a blood vessel wall of the uterine artery 106, and the proximal bent portion 25 is in contact with and stably held by a blood vessel wall on a side opposite to an entrance portion of the uterine artery 106 of a blood vessel wall of the internal iliac artery 105. Accordingly, the distal bent portion 27 and/or the distal linear portion 28 of the catheter 1 can be engaged with the uterine artery 106 in a favorable manner, and positions thereof can be easily maintained. The distal bent portion 27 and/or the distal linear portion 28 may or may not be in contact with the blood vessel wall of the uterine artery 106.

Thereafter, the treatment catheter 5, which is longer than the catheter 1, is inserted into the lumen 21 of the catheter 1 from the hub 3. The surgeon can easily insert the treatment catheter 5 into the uterine artery 106 by protruding the treatment catheter 5 from the distal end of the catheter 1. Thereafter, the surgeon can release an embolic agent and/or a drug solution for occluding the uterine fibroid 111 of the uterus 110 through the treatment catheter 5. The catheter 1 relaxes a reaction generated by releasing the embolic agent, the drug solution, or the like from the treatment catheter 5. Further, the catheter 1 can apply a backup force to the treatment catheter 5 to hold the treatment catheter 5 at a desired position. Therefore, the distal end of the catheter 1 can be prevented from coming out of the entrance of the uterine artery 106 by a reaction generated when the treatment catheter 5 such as a microcatheter is inserted into the lumen 21 of the catheter 1 or when the embolic agent, the drug solution, or the like is released. Note that a medical device to be inserted into the catheter 1 may not be the treatment catheter 5. The surgeon may release the embolic agent and/or the drug solution from the catheter 1 instead of the treatment catheter 5.

Alternatively, as shown in FIG. 6, when the internal iliac artery 105 is short, the surgeon can use the bending of the shaped portion 24 to direct the distal end of the catheter 1 toward the entrance of the uterine artery 106, attach the distal bent portion 27 to the internal iliac artery 105, and attach the proximal bent portion 25 to the common iliac artery 104. In this state, the distal end of the catheter 1 is prevented from coming out of the entrance of the uterine artery 106 by the reaction generated when the treatment catheter 5 such as a microcatheter is inserted into the lumen 21 of the catheter 1 or when the embolic agent, the drug solution, or the like is released.

Alternatively, the distal bent portion 27 may be engaged with a uterine artery ascending branch, the embolic agent may be injected toward a peripheral artery, and the surgeon may release the embolic agent and/or the drug solution by using the catheter 1 and the treatment catheter 5, or may release the embolic agent and/or the drug solution by using the catheter 1 alone.

Note that the catheter 1 may be used to treat benign prostatic hyperplasia of a prostate 120 of a male patient. In this case, an operation of the catheter 1 until the catheter 1 is inserted into the internal iliac artery 105 is the same as the operation of the catheter 1 in the treatment of the uterine fibroid 111. The guide wire 6 and the catheter 1 pass through the internal iliac artery 105 and reach the vicinity of an entrance of the prostate artery 107. The prostate artery 107 is an artery that nourishes the prostate 120, and can be, for example, an inferior vesical artery, a middle rectal artery, an internal pudendal artery, or an obturator artery. After the guide wire 6 and the catheter 1 reach the vicinity of the entrance of the prostate artery 107, the surgeon retracts the guide wire 6 as necessary to accommodate the guide wire 6 in the lumen 21 of the catheter 1. Accordingly, as shown in FIG. 7, the portion of the shaped portion 24 into which the guide wire 6 is not inserted attempts to restore the original bent shape. The surgeon can use the bending of the shaped portion 24 to direct the distal end of the catheter 1 to the entrance of the prostate artery 107, and easily insert the distal end into the prostate artery 107. In this state, the distal bent portion 27 is bent to the same side as the proximal bent portion 25. Therefore, the most distal end of the catheter 1 is more likely to be directed to the prostate artery 107 bifurcating from the internal iliac artery 105, which is thinner than the internal iliac artery 105. Therefore, the distal bent portion 27 and/or the distal linear portion 28 of the catheter 1 are in contact with a blood vessel wall of the prostate artery 107, and the proximal bent portion 25 is in contact with and stably held by a blood vessel wall on a side opposite to an entrance portion of the prostate artery 107 of a blood vessel wall of the internal iliac artery 105. Accordingly, the distal bent portion 27 and/or the distal linear portion 28 of the catheter 1 can be engaged with the prostate artery 107 in a favorable manner, and positions thereof can be easily maintained.

Thereafter, the treatment catheter 5, which is longer than the catheter 1, is inserted into the lumen 21 of the catheter 1 from the hub 3. The surgeon can easily insert the treatment catheter 5 into the prostate artery 107 by protruding the treatment catheter 5 from the distal end of the catheter 1. Thereafter, the surgeon can release an embolic agent and/or a drug solution that reduces a blood flow to the prostate via the treatment catheter 5. According to the catheter 1 of the present embodiment, the distal end of the catheter 1 can be prevented from coming out of the entrance of the prostate artery 107 by a reaction generated when the treatment catheter 5 such as a microcatheter is inserted into the lumen 21 of the catheter 1 or when the embolic agent, the drug solution, or the like is released. Note that when the internal iliac artery 105 is short, the surgeon can use the bending of the shaped portion 24 to direct the distal end of the catheter 1 toward the entrance of the prostate artery 107, attach the distal bent portion 27 to the internal iliac artery 105, and attach the proximal bent portion 25 to the common iliac artery 104. Note that a medical device to be inserted into the catheter 1 may not be the treatment catheter 5. The surgeon may release the embolic agent and/or the drug solution from the catheter 1 instead of the treatment catheter 5.

As described above, the catheter 1 according to the present embodiment is the catheter 1 for uterine artery engagement including a tube shaped body communicating from a proximal end to a distal end. The tube shaped body 2 includes, at a distal portion thereof, the shaped portion 24 shaped in a manner of being bent on substantially the same plane X. The shaped portion 24 includes the proximal bent portion 25 bent to define the first angle θ1, the distal bent portion 27 defining the second angle θ2 distal of the proximal bent portion 25 and bent to the same side as the proximal bent portion 25, the intermediate linear portion 26 that is linear and is disposed between the proximal bent portion 25 and the distal bent portion 27, and the distal linear portion 28 that is linear and is disposed distal of the distal bent portion 27. The first minimum curvature radius R1 which is a smallest curvature radius of an axis of the proximal bent portion 25 is no less than 3 times and no more than 11 times a second minimum curvature radius R2 which is a smallest curvature radius of an axis of the distal bent portion 27.

In the catheter 1 configured as described above, the proximal bent portion 25 is in contact with the internal iliac artery 105, and the distal bent portion 27 that is sharply bent to the same direction as the proximal bent portion 25 with a curvature radius smaller than that of the proximal bent portion 25 and/or the distal linear portion 28 disposed distal of the distal bent portion 27 are easily directed toward the uterine artery 106 and engaged with the uterine artery 106. Therefore, it is easy for the catheter 1 to reach the uterine artery 106 from the radial artery 100 and be engaged with the uterine artery 106.

Further, the catheter 1 according to the present embodiment is the catheter 1 for prostate artery engagement including the tube shaped body 2 communicating from a proximal end to a distal end. The tube shaped body 2 includes, at a distal portion thereof, the shaped portion 24 shaped in a manner of being bent on substantially the same plane X. The shaped portion 24 includes the proximal bent portion 25 bent so as to define the first angle θ1, the distal bent portion 27 defining the second angle θ2 distal of the proximal bent portion 25 and bent to the same side as the proximal bent portion 25, the intermediate linear portion 26 that is linear and is disposed between the proximal bent portion 25 and the distal bent portion 27, and the distal linear portion 28 that is linear and is disposed distal of the distal bent portion 27. The first minimum curvature radius R1 which is a smallest curvature radius of an axis of the proximal bent portion 25 is no less than 3 times and no more than 11 times the second minimum curvature radius R2 which is a smallest curvature radius of an axis of the distal bent portion 27.

In the catheter 1 configured as described above, the proximal bent portion 25 is in contact with the internal iliac artery 105, and the distal bent portion 27 that is sharply bent to the same direction as the proximal bent portion 25 with a curvature radius smaller than that of the proximal bent portion 25 and/or the distal linear portion 28 disposed distal of the distal bent portion 27 are easily directed toward the prostate artery 107 and engaged with the prostate artery 107. Therefore, it is easy for the catheter 1 to reach the prostate artery 107 from the radial artery and be engaged with the prostate artery 107.

Further, the first minimum curvature radius R1 may be 25 mm, the second minimum curvature radius R2 may be 5 mm, the first minimum curvature radius R1 which is the smallest curvature radius of the axis of the proximal bent portion 25 may be 5 times the second minimum curvature radius R2 which is the smallest curvature radius of the axis of the distal bent portion, a length along the axis of the intermediate linear portion 26 may be 16 mm, and a length along the axis of the distal linear portion 28 may be 3 mm. Therefore, it is easy for the catheter 1 to reach the uterine artery 106 or the prostate artery 107 from the radial artery 100 and be engaged with the uterine artery 106 or the prostate artery 107.

The catheter according to the invention may be used in a treatment method using the catheter 1 including the tube shaped body 2 communicating from the proximal end to the distal end. Such a treatment method not falling under the scope of the claims includes: inserting, into a blood vessel from a radial artery, the catheter 1 in which the tube shaped body 2 includes, at the distal portion thereof, the shaped portion 24 shaped in a manner of being bent on substantially the same plane X, the shaped portion 24 includes the proximal bent portion 25 bent to define the first angle θ1, the distal bent portion 27 defining the second angle θ2 distal of the proximal bent portion 25 and bent to the same side as the proximal bent portion 25, the intermediate linear portion 26 that is linear and is disposed between the proximal bent portion 25 and the distal bent portion 27, and the distal linear portion 28 that is linear and is disposed distal of the distal bent portion 27, and the minimum curvature radius R1 of the proximal bent portion 25 is no less than 3 times and no more than 11 times the minimum curvature radius R2 of the distal bent portion 27; and engaging the shaped portion 24 with a blood vessel more peripheral than the internal iliac artery 105.

In the treatment method configured as described above, the proximal bent portion 25 is brought into contact with the internal iliac artery 105 to be stably held by the blood vessel wall, and the distal bent portion 27 that is sharply bent to the same direction as the proximal bent portion 25 with the curvature radius smaller than that of the proximal bent portion 25 and/or the distal linear portion 28 disposed distal of the distal bent portion 27 are easily engaged with the blood vessel more peripheral than the internal iliac artery 105. Therefore, it is easy for the surgeon to perform the treatment in a favorable manner by causing the catheter 1 to reach and be engaged with the blood vessel more peripheral than the internal iliac artery 105 from the radial artery 100.

In the treatment method, the radial artery 100 into which the catheter 1 is to be inserted may be the distal radial artery. Accordingly, it is easy for the surgeon to perform the treatment in a favorable manner by causing the catheter 1 to reach and be engaged with the blood vessel more peripheral than the internal iliac artery 105 in a less invasive manner than in a case of inserting the catheter 1 from the conventional radial artery.

The blood vessel that is disposed on the peripheral side relative to the internal iliac artery 105 to be engaged with the shaped portion 24 may be the uterine artery 106. Accordingly, the surgeon can treat the uterus 110 in a favorable manner by using the catheter 1 inserted into the blood vessel from the radial artery 100.

A treatment target may be the uterine fibroid 111. Accordingly, the surgeon can treat the uterine fibroid 111 in a favorable manner by using the catheter 1 inserted into the blood vessel from the radial artery 100.

The blood vessel that is disposed on the peripheral side relative to the internal iliac artery 105 to be engaged with the shaped portion 24 may be the prostate artery 107. Accordingly, the surgeon can treat the prostate 120 (for example, treat the benign prostatic hyperplasia) in a favorable manner by using the catheter 1 inserted into the blood vessel from the radial artery 100.

A treatment target is the enlarged prostate 120 due to the benign prostatic hyperplasia. Accordingly, the surgeon can treat the benign prostatic hyperplasia in a favorable manner by using the catheter 1 inserted into the blood vessel from the radial artery 100.

Note that the invention is not limited to the embodiment described above, and various modifications can be made by those skilled in the art within a scope of the technical idea of the invention.

Note that the present application is based on Japanese Patent Application No. 2020-95742 filed on June 1, 2020 and Japanese Patent Application No. 2020-197870 filed on November 30, 2020.

### Reference Signs List

1 catheter
2 tube shaped body
21 lumen
23 main body portion
24 shaped portion
25 proximal bent portion
26 intermediate linear portion
27 distal bent portion
28 distal linear portion
100 radial artery
101 subclavian artery
102 aortic arch
103 descending aorta
104 common iliac artery
105 internal iliac artery
106 uterine artery
107 prostate artery
110 uterus
111 uterine fibroid
120 prostate
A first axis
B second axis
C third axis
R1 first minimum curvature radius
R2 second minimum curvature radius
X plane
θ1 first angle
θ2 second angle
θ3 third angle

## Claims

1. A catheter (1) for uterine artery or prostate artery engagement comprising:
a tube shaped body (2) communicating from a proximal end to a distal end,
wherein the tube shaped body (2) includes, at a distal portion (27) thereof, a shaped portion (24) shaped in a manner of being bent on substantially the same plane (X),
the shaped portion (24) includes:
a proximal bent portion (25) bent to define a first angle (θ1);
a distal bent portion (27) defining a second angle distal (θ2) of the proximal bent portion (25) and bent to the same side as the proximal bent portion (25);
an intermediate linear portion (26) that is linear and is disposed between the proximal bent portion (25) and the distal bent portion (27); and
a distal linear portion (28) that is linear and is disposed distal of the distal bent portion (27), and
a first minimum curvature radius (R1) which is a smallest curvature radius of an axis of the proximal bent portion (25) is no less than 3 times and no more than 11 times a second minimum curvature radius (R2) which is a smallest curvature radius of an axis of the distal bent portion (27),
a length (L3) along the axis of the distal bent portion (27) is 1 mm to 10 mm, and
the catheter (1) is coated with a lubricating coating in a proximal direction along an axis from a distal end opening portion (22) in a range of 500 mm or less mm.

2. The catheter according to claim 1,
wherein the first minimum curvature radius (R1) is 25 mm, the second minimum curvature radius (R2) is 5 mm, the first minimum curvature radius (R1) is 5 times the second minimum curvature radius (R2), a length (L2) along an axis of the intermediate linear portion (26) is 16 mm, and a length (L4) along an axis of the distal linear portion (28) is 3 mm.

## Patentansprüche

1. Katheter (1) zum Eingriff in die Gebärmutterarterie oder die Prostataarterie, umfassend:
einen röhrenförmigen Körper (2), der von einem proximalen Ende zu einem distalen Ende in Verbindung steht,
wobei der röhrenförmige Körper (2), an einem distalen Abschnitt (27) davon, einen geformten Abschnitt (24) aufweist, der in einer Weise geformt ist, dass er im Wesentlichen in derselben Ebene (X) gebogen ist,
wobei der geformte Abschnitt (24) umfasst:
einen proximalen gebogenen Abschnitt (25), der so gebogen ist, dass er einen ersten Winkel (θ1) definiert;
einen distalen gebogenen Abschnitt (27), der einen zweiten Winkel (θ2) distal des proximalen gebogenen Abschnitts (25) definiert und zur gleichen Seite wie der proximale gebogene Abschnitt (25) gebogen ist;
einen mittleren linearen Abschnitt (26), der linear ist und zwischen dem proximalen gebogenen Abschnitt (25) und dem distalen gebogenen Abschnitt (27) angeordnet ist; und
einen distalen linearen Abschnitt (28), der linear ist und distal von dem distalen gebogenen Abschnitt (27) angeordnet ist, und
ein erster minimaler Krümmungsradius (R1), welcher der kleinste Krümmungsradius einer Achse des proximalen gebogenen Abschnitts (25) ist, nicht weniger als das 3-fache und nicht mehr als das 11-fache eines zweiten minimalen Krümmungsradius (R2) beträgt, welcher der kleinste Krümmungsradius einer Achse des distalen gebogenen Abschnitts (27) ist,
eine Länge (L3) entlang der Achse des distalen gebogenen Abschnitts (27) 1 mm bis 10 mm beträgt, und
der Katheter (1) in einer proximalen Richtung entlang einer Achse von einem distalen Endöffnungsabschnitt (22) in einem Bereich von 500 mm oder weniger mit einer Gleitbeschichtung beschichtet ist.

2. Katheter nach Anspruch 1,
wobei der erste minimale Krümmungsradius (R1) 25 mm beträgt, der zweite minimale Krümmungsradius (R2) 5 mm beträgt, der erste minimale Krümmungsradius (R1) das Fünffache des zweiten minimalen Krümmungsradius (R2) beträgt, eine Länge (L2) entlang einer Achse des mittleren linearen Abschnitts (26) 16 mm beträgt und eine Länge (L4) entlang einer Achse des distalen linearen Abschnitts (28) 3 mm beträgt.

## Revendications

1. Cathéter (1) pour logement dans une artère utérine ou une artère prostatique comprenant :
un corps en forme de tube (2) communiquant d'une extrémité proximale à une extrémité distale,
dans lequel le corps en forme de tube (2) comporte, au niveau d'une partie distale (27) de celui-ci, une partie moulée (24) de manière à être courbée sur sensiblement le même plan (X),
la partie moulée (24) comporte :
une partie courbée proximale (25) courbée pour définir un premier angle (θ1) ;
une partie courbée distale (27) définissant un second angle distal (θ2) de la partie courbée proximale (25) et courbée du même côté que la partie courbée proximale (25) ;
une partie linéaire intermédiaire (26) qui est linéaire et est disposée entre la partie courbée proximale (25) et la partie courbée distale (27) ; et
une partie linéaire distale (28) qui est linéaire et disposée distalement par rapport à la partie courbée distale (27), et
un premier rayon de courbure minimal (R1) qui est un rayon de courbure le plus petit d'un axe de la partie courbée proximale (25) n'est pas inférieur à 3 fois et non supérieur à 11 fois un second rayon de courbure minimal (R2) qui est un rayon de courbure le plus petit d'un axe de la partie courbée distale (27),
une longueur (L3) le long de l'axe de la partie courbée distale (27) est de 1 mm à 10 mm, et le cathéter (1) est revêtu d'un revêtement lubrifiant dans une direction proximale le long d'un axe à partir d'une partie d'ouverture d'extrémité distale (22) dans une plage de 500 mm ou moins.

2. Cathéter selon la revendication 1,
dans lequel le premier rayon de courbure minimal (R1) est de 25 mm, le second rayon de courbure minimal (R2) est de 5 mm, le premier rayon de courbure minimal (R1) est égal à 5 fois le second rayon de courbure minimal (R2), une longueur (L2) le long d'un axe de la partie linéaire intermédiaire (26) est de 16 mm, et une longueur (L4) le long d'un axe de la partie linéaire distale (28) est de 3 mm.
